(19) Europäisches Patentamt
European Patent Office
Office européen des brevets

(11) **EP 4 736 798 A1**

(12) **EUROPEAN PATENT APPLICATION**

(43) Date of publication:
**06.05.2026 Bulletin 2026/19**

(51) International Patent Classification (IPC):
***A61B 18/14*** *(2006.01)*

(21) Application number: **25212655.2**

(22) Date of filing: **31.10.2025**

(52) Cooperative Patent Classification (CPC):
**A61B 18/14;** A61B 2018/0022

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH LA MA MD TN**

(30) Priority: **01.11.2024 US 202418935326**

(71) Applicant: **Biosense Webster (Israel) Ltd.**
**2066717 Yokneam (IL)**

(72) Inventors:
• **ADAME, Alejandro**
  **Irvine, 92618 (US)**
• **RAMPA, Sreekanth Reddy**
  **Irvine, 92618 (US)**

(74) Representative: **Carpmaels & Ransford LLP**
**One Southampton Row**
**London WC1B 5HA (GB)**

(54) **ELECTROPHYSIOLOGY CATHETER SYSTEM WITH EXPANDABLE ABLATION MEMBER FOR COMPLETE CIRCUMFERENTIAL ABLATION OF TUBULAR REGIONS AND RELATED METHODS**

(57) A catheter system is for use with a catheter with an expandable balloon member of a conic configuration with a maximum radius delineating between a distal portion and a proximal portion, the balloon member including a plurality of circumferential flexible circuits disposed thereon in the distal portion of the balloon member, the balloon member defining a longitudinal axis, each ablation flexible circuit extending circumferentially around the longitudinal axis with a different respective radius at a different respective location along the longitudinal axis wherein a circumferential flexible circuit with a conforming radius for contact with tissue is selectively energized to create a complete circumferential lesion around tubular region of the heart.

*FIG. 3*

EP 4 736 798 A1

**Description**

BACKGROUND

Cardiac arrhythmia, such as atrial fibrillation, occurs when regions of cardiac tissue abnormally conduct electric signals to adjacent tissue, thereby disrupting the normal cardiac cycle and causing asynchronous rhythm. Sources of undesired signals may be located in or near an atria or a ventricle. Unwanted signals may be conducted elsewhere through heart tissue where they can initiate or continue arrhythmia.

Procedures for treating arrhythmia include surgically disrupting the origin of the signals causing the arrhythmia, as well as disrupting the conducting pathway for such signals. By mapping the electrical properties of the endocardium and the heart volume, and selectively ablating cardiac tissue by application of energy, it may be possible to cease or modify the propagation of unwanted electrical signals from one portion of the heart to another. The ablation process may destroy the unwanted electrical pathways by formation of non-conducting regions of tissue.

In this two-step procedure, which includes mapping followed by ablation, electrical activity at points in the heart may be sensed and measured by advancing a first or mapping catheter containing one or more electrical sensors into the heart and acquiring data at multiple points. These data may then be utilized to select the target areas at which ablation is to be performed by a second or ablation catheter.

During ablation, RF current is applied to a first electrode of the ablation catheter and current flows through the media that surrounds it, i.e., blood and tissue, toward a second electrode which may be another electrode on the catheter or an external skin patch reference electrode. The distribution of current depends on the amount of electrode surface in contact with the tissue as compared to blood, which has a higher conductivity than the tissue. Heating of tissue occurs due to its electrical resistance. The tissue is heated sufficiently to cause cellular destruction in the target tissue resulting in formation of a lesion which is electrically non-conductive. The lesion may be formed in tissue contacting the electrode or in adjacent tissue. During this process, heating of the electrode also occurs as a result of conduction from the heated tissue to the electrode itself.

Using a multi-electrode catheter for irreversible electroporation (IRE) has been previously proposed in patent literature. For example, PCT International Publication WO 2018/191149 describes electroporation systems and methods of energizing a catheter for delivering electroporation. A catheter for delivery electroporation includes a distal section and an electrode assembly. The distal section is configured to be positioned in a vein within a body. The vein defines a central axis. The electrode assembly is coupled to the distal section and includes a structure and a plurality of electrodes distributed thereabout. The structure is configured to at least partially contact the vein. Each of the electrodes is configured to be selectively energized. In an embodiment, each electrode is individually wired such that it can be selectively paired or combined with any other electrode to act as a bipolar or a multi-polar electrode.

As another example, U.S. Patent No. 8,295,902 describes a tissue electrode assembly that includes a membrane configured to form an expandable, conformable body that is deployable in a patient. The assembly further includes a flexible circuit positioned on a surface of the membrane. An electrically-conductive electrode covers at least a portion of the flexible circuit and a portion of the surface of the membrane not covered by the flexible circuit, wherein the electrically-conductive electrode is foldable upon itself with the membrane to a delivery conformation having a diameter suitable for minimally-invasive delivery of the assembly to the patient. In an embodiment, a pattern of multiple electrodes deposited on the membrane can collectively create a large electrode array of energy-transmitting elements.

As a further example, Chinese Patent No. CN 112545643 describes a balloon catheter and an ablation system, wherein the electrode is arranged at the far end of the catheter and comprises an electrode near end, an electrode main body and an electrode far end which are sequentially connected along the extension direction of the catheter, the electrode main body is of a net structure, at least one of the electrode near end and the electrode far end is movably connected with the catheter, and the electrode main body is configured to be switched between a contraction state and an expansion state along with the movement of the electrode near end and/or the electrode far end along the catheter. The balloon catheter comprises a balloon and the electrode, and is used for supporting the electrode main body to keep an expanded state when the balloon is expanded so as to enable the electrode main body to be attached to a preset object. The balloon moves to the far end of the electrode through self expansion or balloon expansion to apply pressure stress to the electrode main body, so that the electrode main body is attached to a preset object more stably, continuously and completely, and the ablation efficiency is improved. Further, a cooling medium may be provided into the balloon to improve safety during the ablation stage.

U.S. Patent No. 8992519 describes a method for forming an ablation catheter having a balloon at a distal end of the catheter. The method includes applying a band of conductive material to an outer surface of the balloon. The band of conductive material is adapted to provide one or more electrodes for radio frequency ablation therapy. A distal end of lead is connected to the band of conductive material. The balloon is inverted, so that the inverted balloon includes the band of conductive material on an inside surface. According to various embodiments, the balloon includes semi-permeable or hydro-able membrane.

U.S. Patent No. 9060756 describes a method of ablating body tissue that includes: (a) locating an inflatable balloon portion of a cryotherapy balloon catheter at a treatment site internal to a patient's body, and inflating the inflatable ballon portion; (b) employing electrodes that are disposed on an expandable surface of the inflatable balloon portion to electrically characterize body tissue at the treatment site; (c) ablating the body tissue by supplying a cryotherapy agent to the inflatable balloon portion to cool the body tissue to a therapeutic temperature 9d) employ the electrodes to determine whether the ablation caused desired electrical changes in the body tissue; and (e) repeating (c) and (d) when it is determined that the ablating did not cause the desire electrical changes.

Ablation of a tubular region of the heart, including a pulmonary vein can pose unique challenges. Pulmonary vein diameters at the ostia can vary within any one patient's heart but pulmonary vein diameters can also vary between different patients. For example, diameter and cross-sectional area of the left superior pulmonary vein can be significantly larger in men than in women.

Conventional ring electrode with exposed electrode inner diameter regions may experience loss of energy during ablation and become breeding grounds for microbubbles during ablation. Conventional discrete electrodes may also generate inconsistent electric fields between the electrodes creating weak spots for tissue ablation. Size of the electrode ablation assembly is also a concern, especially for pulsed field ablation which benefit from full electrode contact, as larger electrode ablation assembly can be difficult to maneuver and position in tubular regions of the heart.

Applicants recognized there is a need to provide a catheter with an ablation end effector that can ablate tissue of a tubular region completely circumferentially in conformity with tubular regions of different sizes without space gaps or weakened electric fields. Applicants also recognize there is a need for a true "single shot" ablation catheter with larger surface area per electrode for pulsed field ablation (PFA) to cause irreversible tissue electroporation (IRE), as larger surface area provides better dissipation of latent heat in tissue due to joule heating. Applicants further recognize there is a need for ablation end effector with "one-sided" electrodes without an internal region from which microbubbles can form or ablation energy can be lost to blood.

BRIEF DESCRIPTION OF THE DRAWINGS

While the specification concludes with claims which particularly point out and distinctly claim this technology, it is believed this technology will be better understood from the following description of certain examples taken in conjunction with the accompanying drawings, in which like reference numerals identify the same elements.

FIG. 1 is a pictorial illustration of an electrophysiology catheter system for sensing performing ablation on tissue in a tubular region of the heart, in accordance with an embodiment of the invention.

FIG. 2A is a perspective view of an ablation end effector of the catheter of FIG. 1, in an expanded configuration in accordance with an embodiment of the invention.

FIG. 2B is a detailed view of an impedance sensor of FIG. 2A, in accordance with an embodiment of the invention.

FIG. 3 is side view of the end effector of FIG. 2A, in a collapsed configuration, in accordance with an embodiment of the invention.

FIG. 4A is a side view of the end effector of FIG. 2A situated a small ostium.

FIG. 4B is a side view of the end effector of FIG. 2A situated in a larger ostium.

FIG. 5 is a top plan view of circumferential flexible circuits with planar form prior to affixation to the ablation balloon member.

FIG. 6 is a side cross-sectional view of a circumferential flexible circuit on a balloon member in contact with tissue of a tubular region during ablation in forming a lesion.

FIG. 7A is plan view of a nontissue-facing surface of a circumferential flexible circuit, in accordance with another embodiment.

FIG. 7B is a plan view of a tissue-facing surface of the circumferential flexible circuit of FIG. 7A.

FIG. 8A is a plan view of a force sensitive resistor, in accordance with an embodiment.

FIG. 8B is an exploded view of the force sensitive resistor of FIG. 8A.

FIG. 9A is a top plan view of a tri-axes position sensor, according to an embodiment.

FIG. 9B is a side view of the tri-axes position sensor of FIG. 9A.

FIG. 9C is a perspective view of the tri-axes position sensor of FIG. 9A in a coiled configuration as mounted on a shaft.

FIG. 10A is a side view of an ablation end effector in an expanded configuration in accordance with another embodiment of the invention.

FIG. 10B is a front perspective view of the ablation end effector of FIG. 10A in transition between a collapsed configuration and an expanded configuration.

FIG. 10C is a side view of the ablation end effector of FIG. 10A in a collapsed configuration, with a second catheter extending therethrough, in accordance with an embodiment of the invention.

FIG. 11 is a flowchart illustrating a method of ablating using the catheter system of FIG. 1, in accordance with an embodiment of the present invention.

DETAILED DESCRIPTION FOR MODES OF CARRYING OUT THE INVENTION

The following detailed description should be read with reference to the drawings, in which like elements in different drawings are identically numbered. The drawings, which are not necessarily to scale, depict selected embodiments and are not intended to limit the scope of the invention. The detailed description illustrates by way of example, not by way of limitation, the principles of the invention. This description will clearly enable one skilled in the art to make and use the invention, and describes several embodiments, adaptations, variations, alternatives and uses of the invention, including what is presently believed to be the best mode of carrying out the invention.

Any one or more of the teachings, expressions, versions, examples, etc. described herein may be combined with any one or more of the other teachings, expressions, versions, examples, etc. that are described herein. The following-described teachings, expressions, versions, examples, etc. should therefore not be viewed in isolation relative to each other. Various suitable ways in which the teachings herein may be combined will be readily apparent to those skilled in the pertinent art in view of the teachings herein. Such modifications and variations are intended to be included within the scope of the claims.

Example end effectors are illustrated and disclosed herein which are generally planar and include multiple electrodes that can be configured for mapping and/or ablation. The end effectors can be joined to a shaft with additional catheter components to form a mapping and/or ablation catheter through processes disclosed herein and processes similar to those known by a person skilled in the pertinent art. The example end effectors illustrated herein include variations and features that are combinable to form additional end effector designs as understood by a person skilled in the pertinent art.

The following detailed description should be read with reference to the drawings, in which like elements in different drawings are identically numbered. The drawings, which are not necessarily to scale, depict selected embodiments and are not intended to limit the scope of the invention. The detailed description illustrates by way of example, not by way of limitation, the principles of the invention. This description will clearly enable one skilled in the art to make and use the invention, and describes several embodiments, adaptations, variations, alternatives and uses of the invention, including what is presently believed to be the best mode of carrying out the invention.

As used herein, the terms "about" or "approximately" for any numerical values or ranges indicate a suitable dimensional tolerance that allows the part or collection of components to function for its intended purpose as described herein. More specifically, "about" or "approximately" may refer to the range of values ±20% of the recited value, e.g. "about 90%" may refer to the range of values from 71% to 110%. In addition, as used herein, the terms "patient," "host," "user," and "subject" refer to any human or animal subject and are not intended to limit the systems or methods to human use, although use of the subject invention in a human patient represents a preferred embodiment. As well, the term "proximal" indicates a location closer to the operator or physician whereas "distal" indicates a location further away to the operator or physician.

As discussed herein, vasculature of a "patient," "host," "user," and "subject" can be vasculature of a human or any animal. It should be appreciated that an animal can be a variety of any applicable type, including, but not limited thereto, mammal, veterinarian animal, livestock animal or pet type animal, etc. As an example, the animal can be a laboratory animal specifically selected to have certain characteristics similar to a human (e.g., rat, dog, pig, monkey, or the like). It should be appreciated that the subject can be any applicable human patient, for example.

As discussed herein, "operator" can include a physician, doctor, surgeon, technician, scientist, or any other individual or delivery instrumentation associated with delivery of a multi-electrode catheter for the treatment of drug refractory atrial fibrillation to a subject.

As discussed herein, the term "ablate" or "ablation", as it relates to the devices and corresponding systems of this disclosure, refers to components and structural features configured to reduce or prevent the generation of erratic cardiac signals in the cells by utilizing non-thermal energy, such as irreversible electroporation (IRE), referred throughout this disclosure interchangeably as pulsed electric field (PEF) and pulsed field ablation (PFA). Ablating or ablation as it relates to the devices and corresponding systems of this disclosure is used throughout this disclosure in reference to non-thermal ablation of cardiac tissue for certain conditions including, but not limited to, arrhythmias, atrial flutter ablation, pulmonary vein isolation, supraventricular tachycardia ablation, and ventricular tachycardia ablation. The term "ablate" or "ablation" also includes known methods, devices, and systems to achieve various forms of bodily tissue ablation as understood by a person skilled in the relevant art.

As discussed herein, the terms "bipolar" and "unipolar" when used to refer to ablation schemes describe ablation schemes which differ with respect to electrical current path and electric field distribution. "Bipolar" refers to ablation scheme utilizing a current path between two electrodes that are both positioned at a treatment site; current density and electric flux density is typically approximately equal at each of the two electrodes. "Unipolar" refers to ablation scheme utilizing a current path between two electrodes where one electrode having a high current density and high electric flux density is positioned at a treatment site, and a second electrode having comparatively lower current density and lower electric flux density is positioned remotely from the treatment site.

As discussed herein, the terms "tubular" and "tube" are to be construed broadly and are not limited to a structure that is a right cylinder or strictly circumferential in cross-section or of a uniform cross-section throughout its length. For example, the tubular structures are generally illustrated as a substantially right cylindrical structure. However, the tubular structures may have a tapered or curved outer surface without departing from the scope of the present disclosure.

In an electrophysiology system, electrical activity within the heart is detected using a catheter with two or more electrodes for bipolar mapping. Electrical activity within the heart is detected using a first electrode configured for sensing both local activation energy (near-field signals) at the point of contact with heart tissue and far field activation energy (far-field signals) received by the electrode through the blood. In close proximity to the first electrode, the second electrode is configured to receive approximately the same far-field signals but not the local activation energy (near-fields signals). A suitable signal processing unit processes the signals received by both the first and second electrodes and by subtracting the far-field signals detected by the second electrode from the near-and far-field signals detected by the first electrode, near-field signals can be more accurately determined.

The catheter typically also comprises a location sensor, e.g., an electromagnetic position sensor. Suitable electromagnetic sensors are described in U.S. Patent Nos. 5,443,489, 5,480,422, 5,546,951, 5,568,809 and 5,391,199, the disclosures of which are incorporated herein by reference. In some embodiments, to use the electromagnetic sensor, the patient is placed in a magnetic field generated, for example, by situating under the patient a pad containing coils for generating magnetic field(s). A reference electromagnetic sensor is fixed relative to the patient, e.g., taped to the patient's back, and the catheter with the electromagnetic location sensor is advanced into the patient's heart. Each sensor preferably comprises three small coils which in the magnetic field(s) generate electrical signals indicative of their position in the magnetic field(s). Signals generated by both the fixed reference sensor and the sensor in the catheter in the heart are processed to ascertain a precise location of the sensor in the catheter relative to the reference sensor. Using this technology, a physician can visually map a heart chamber. This mapping is done by advancing the catheter into a heart chamber until a distal tip makes contact with the heart wall. This position is recorded and saved. The distal tip is then moved to another position in contact with the heart wall and again the position is saved. By combining the electromagnetic sensor and sensing electrodes, a physician can simultaneously map the contours or shape of the heart chamber and the electrical activity of the heart and generate 3-D electroanatomical maps for display on a monitor. Errant electrical activities of the patient's heart may therefore be viewed and diagnosed by the physician.

The 3-D electroanatomical map may also be based on an estimated anatomical map. Mapping algorithms that are based on such measurements, such as fast anatomical mapping (FAM), are known in the art. The FAM method may provide a physician with additional mapping capabilities, such as electro-physiological (EP) mapping that may be used for cardiac ablation. During the FAM procedure, a physician navigates the distal end of the catheter to desired locations in the heart to collect anatomical signals therefrom. In principle, the FAM may provide the physician with a surface representing an estimated anatomical mapping of the tissue in question. Point positions on the surface of a heart chamber are drawn using acquired electroanatomical data. This surface will be used by the physician during EP mapping and ablation procedures.

Catheters may also be configured to provide hybrid magnetic-based and impedance-based position sensing in benefitting from both the higher accuracy of magnetic position sensing and the lower cost of impedance-based sensing. In impedance-based active current location (ACL) sensing, impedance is measured between electrodes on the catheter and external skin patch electrodes placed on the patient's body. An electrical signal is applied to the electrodes on the catheter ("active" electrodes) and the resulting voltages and/or currents are measured at the external skin patch electrodes ("return" electrode). In hybrid position sensing, externally-applied magnetic fields are measured by the magnetic field sensor, and accurate position coordinates of the catheter are derived. Currents or voltages from the external skin patch electrodes are also applied, and impedances between the external skin patch electrodes and the catheter electrodes are measured. The dual position measurements are repeated at multiple locations within the body cavity in order to generate a calibration map, correlating the impedance measurements with position coordinates ascertained by the magnetic field sensor. Additional catheters with diagnostic or therapeutic functions may be introduced into the heart, and these additional catheters need not include magnetic field sensors, as impedance measurements taken from electrodes of these additional catheters are correlated with the calibration map in order to determine accurate position coordinates of these additional catheters. Notably, typical values of frequency and amplitude of the ACL signals are on the order of 10 kilohertz (kHz) and 1 millivolts (mV), respectively, whereas the respective values of frequency and amplitude of the ECG signals are on the order of 1 hertz (Hz) and 1 microvolt ($\mu$V).

An ablation system typically comprises a catheter with at least two electrodes coupled to an energy source. One of the electrode is configured as an anode and the other as a cathode. Electrodes may be energized with DC voltages and conduct currents are various frequencies, amplitudes, pulse widths and polarities. When the energy source supplies an energizing potential to an electrode, an electrical current is conducted between the first and second electrodes through patient tissue. Polarity of the electrodes may be reversed by reversing the polarity of the output of the energy source. The electric current supplied by the energy source may comprise pulses or pulse sequences. Each pulse may be monophasic including a single polarity, or biphasic including a first component having a polarity and a second component having an opposite polarity. Pulses may include blended unipolar/bipolar pulses.

Irreversible electroporation (IRE), also called Pulsed Field Ablation (PFA), may be used as an invasive therapeutic modality to kill tissue cells by subjecting them to high-voltage pulses. Specifically, IRE pulses have a potential use to kill myocardium tissue cells in order to treat cardiac arrhythmia. Cellular destruction occurs when the transmembrane potential exceeds a threshold, leading to cell death and thus the development of a tissue lesion. Therefore, of particular interest is the use of high-voltage bipolar electric pulses, e.g., using a selected pair of electrodes in contact with tissue to generate high electric fields, e.g., above a certain threshold to kill tissue cells between the electrodes. Embodiments of the present invention that are described hereinafter use a multi-electrode catheter configured for both mapping and ablation, including RF, PFA and IRE ablation of IRE pulses with typical magnitudes of 1kV - 3kV, operable in both unipolar and bipolar modes.

I. Overview of an Embodiment of an Ablation System

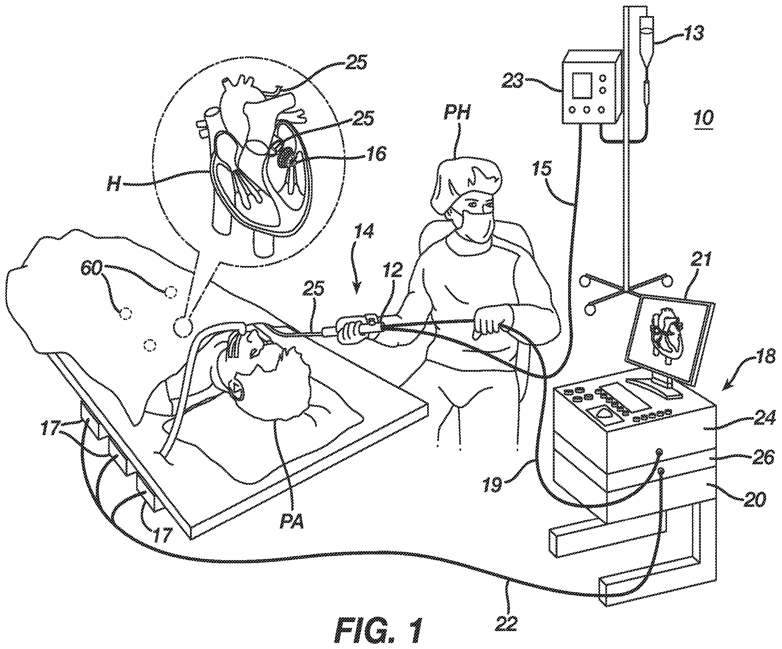

**FIG. 1** shows an embodiment of a medical procedure and associated components of a cardiac ablation system 10. In particular, **FIG. 1** shows a physician (PH) grasping a handle 12 of an ablation catheter assembly 14, with an end effector 16, disposed in a patient PA to ablate tissue in or near the heart H of the patient PA. Ablation catheter assembly 14 is coupled with a guidance and drive system 18 via cable 19. In some embodiments, the ablation catheter assembly 14 is also coupled with a fluid source 13 via a fluid conduit 15. A set of magnetic field generators 17 are positioned underneath the patient PA and are coupled with the guidance and drive system 18 via cable 22.

Guidance and drive system 18 of the illustrated embodiment includes a console 20 and a display 21. The console 20 includes a first driver module 24 and a second driver module 26. First driver module 14 is coupled with ablation catheter assembly 14 via cable 19 and is operable to provide ablation energy to electrodes of end effector 16 as will be described in greater detail below. In some embodiments, the first driver module 24 is also operable to receive position indicative signals from a position sensor (not shown) in the end effector 16. In such embodiments, a processor of the console 20 is also operable to process the position indicative signals from the position sensor to thereby determine the position of the end effector 16 of ablation catheter 28 within the patient PA.

The second driver module 26 is coupled with the field generators 17 via the cable 22. The second driver module 26 is operable to activate field generators 17 to generate alternating magnetic fields around the heart H of the patient PA. For instance, the field generators 17 may include coils that generate alternating magnetic fields in a predetermined working volume that contains the heart H.

Display 21 is coupled with the processor of console 20 and is operable to render images of patient anatomy. Such images may be based on a set of preoperatively or interoperatively obtained images (e.g., a CT or MRI scan, 3-D map, etc.). The views of patient anatomy provided through display 21 may also change dynamically based on signals from the position sensor of the end effector 16. For instance, as the end effector 16 of ablation catheter moves within the patient PA, the corresponding position data from the position sensor may cause the processor of console 20 to update the patient anatomy views in display 21 in real time to depict the regions of patient anatomy around the end effector 16 as the end effector moves within the patient PA. Moreover, the processor of console 20 may drive display 21 to show locations of aberrant conductive tissue sites, as detected via EP mapping with a dedicated mapping catheter (not shown). By way of example only, the processor of console 20 may drive display 21 to superimpose the locations of aberrant conductive tissue sites on the images of the patient's anatomy, such as by superimposing an illuminated dot, a crosshair, or some other form of visual indications of aberrant conductive tissue sites.

The processor of the console 20 may also drive the display 21 to superimpose the current location of end effector 16 on the images of the patient's anatomy, such as by superimposing an illuminated dot, a crosshair, a graphical representation of the end effector 16, or some other form of visual indication. Such a superimposed visual indication may also move within the images of the patient anatomy on display 21 in real time as the physician moves end effector 16 within the patient PA, thereby providing real-time visual feedback to the operator about the position of the end effector 16 within the patient PA as the end effector 16 moves within the patient PA. The images provided through the display 21 may thus effectively provide a video tracking the position of the end effector 16 within the patient PA, without necessarily having an optical instrumentation (i.e., cameras) viewing the end effector 16. In the same view, the display 21 may simultaneously visually indicate the locations of aberrant conductive tissue sites detected through the EP mapping as described herein. The physician PH may thus view the display 21 to observe the real time positioning of the end effector 16 in relation to the mapped aberrant conductive tissue sites and in relation to images of the adjacent anatomical structures in the patient PA.

The fluid source 13 of the illustrated embodiment includes a bag containing saline or some other suitable irrigation fluid. The conduit 15 includes a flexible tube that is further coupled with a pump 23. The pump 23 is positioned along conduit 15 between the fluid source 13 and the ablation catheter assembly 14. In the illustrated embodiment, the pump 23 includes a peristaltic pump that is operable to selectively drive fluid from the fluid source 13 to the ablation catheter assembly 14. Alternatively, the pump 23 may take any other suitable form.

In some embodiments, the conduit 15, the fluid source 13, and the pump 23 are omitted entirely. In embodiments where these components are included, the end effector 16 may be configured to communicate irrigation fluid from the fluid source 13 to the target site in the patient. Such irrigation may be provided in accordance with the teachings of any of the various patent references cited herein; or in any other suitable fashion as will be apparent to those skilled in the art in view of the teachings herein.

II. Expandable Assembly with Circumferential Flex Circuit Bands

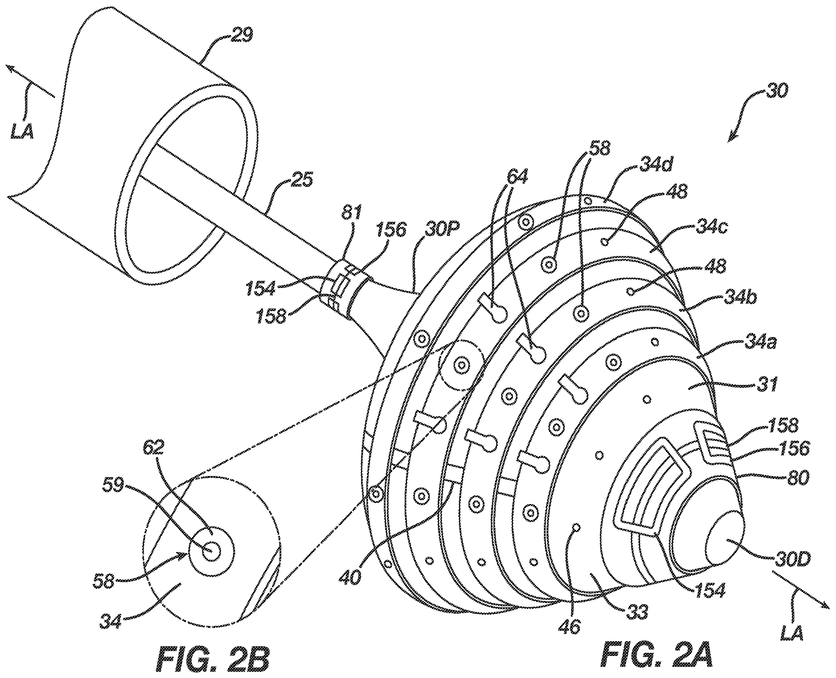

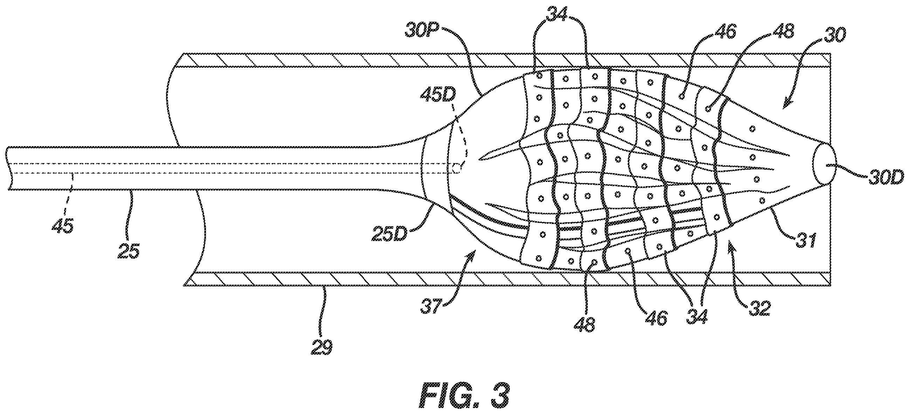

**FIG. 2A** shows the end effector 16 in greater detail. As shown, the end effector 16 is mounted on a distal end of an elongated flexible shaft 25, a distal section of which may be deflectable in response to deflection mechanisms in the control handle 12 manipulated by the physician PA. The end effector 16 includes an expandable ablation balloon member 30 configured to transition between an expanded configuration **(FIG. 2A)** and a collapsed configuration **(FIG. 3).** The ablation balloon member 30 may be kept in the collapsed configuration as the ablation catheter 28 is inserted into the patient PA via a guiding sheath 29. Once ablation balloon member 30 reaches a target site in the patient, the balloon member 30 may be transitioned to the expanded configuration, for example, when the balloon member 30 is advanced distally past a distal end of the guiding sheath 29. In some embodiments, the ablation catheter 28 is slidably disposed in the guiding sheath 28 and the ablation catheter 28 and the guiding sheath 29 are advanced together into the vasculature (e.g., artery, vein, etc.) of the patient PA until the distal end of the guiding sheath 28 is near a target site in the patient PA. The ablation balloon member 30 may be initially retracted proximally relative to the distal end of the guiding sheath 29 as the combination of the ablation catheter and the guiding sheath are advanced into position. Once reaching the target site, the ablation catheter 28 may be advanced distally relative to the guiding sheath 29, to thereby advance the ablation balloon member 30.

In some embodiments, the ablation balloon member 30 includes a balloon membrane 31 of a biocompatible material, for example, polyethylene terephthalate (PET), polyurethane or PEBAX®. The balloon member 30 extends from the distal end of the shaft 25 which is lumened. An irrigating tubing 45 extends through the lumen and has a distal end 45D generally coterminous with the distal end of the shaft 25 to deliver irrigation fluid into an interior of the balloon member 30.

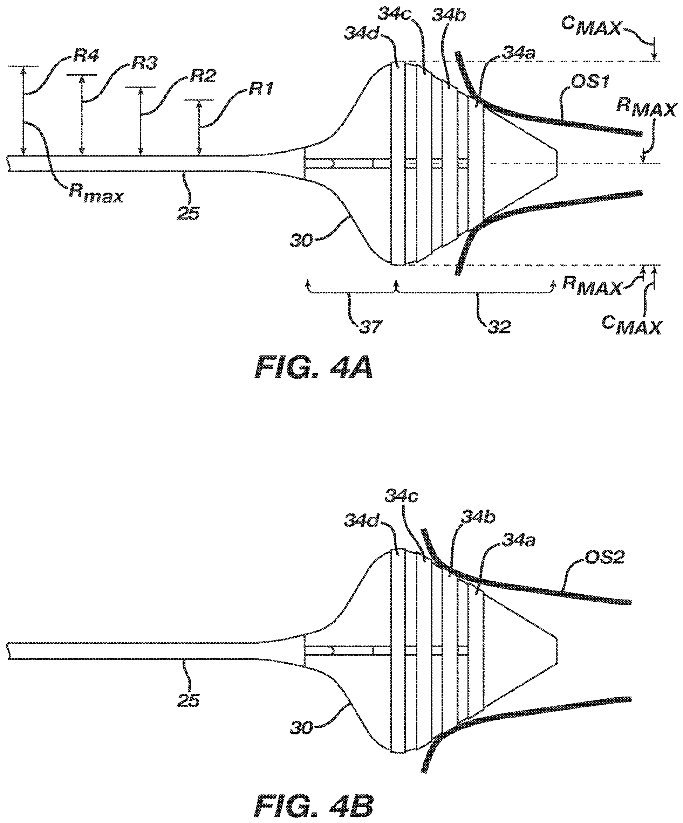

In the illustrated embodiment, the shaft 25 defines a longitudinal axis LA with the balloon member 30 spanning a length L from a proximal end 30P to an atraumatic distal end 30D along the axis LA. With reference to **FIG. 4A,** the balloon member 30 has a distal portion 32 and a proximal portion 37, delineated by a maximum circumference Cmax or maximum radius Rmax of the balloon member (where "circumference" and "radius" are used interchangeably herein with the understanding that $C = 2\Pi R$). In the illustrated embodiment, the distal portion 32 has a conical configuration that is symmetrical about the axis LA, where the conical distal portion 32 is defined by a decreasing radius from the maximum radius Rmax at a predetermined location distal of the proximal end 30P and a minimum radius RMin at the distal end 30D to result in the tapered profile of the balloon member. In the illustrated embodiment, the predetermined location of the maximum radius is about 1/3L from the proximal end 30P and about 2/3L from the distal end 30D, with Rmin ranging between about 0.3Rmax to 0.1Rmax, and preferably Rmin = (0.15) Rmax. In the illustrated embodiment, the taper of the conical distal portion 32 or rate at which the radius decreases is linear. In some embodiments, the distal edge of the most distal circumferential electrode has a diameter of 23 mm with the proximal region of the most proximal circumferential electrode at 32 mm. Notably, the conical distal portion 32 provides the balloon member 30 with an outer surface 33 on which differently-sized circumferential bands 34 of ablation flex circuits, for example, 34a, 34b, 34c, 34d, can be disposed for tissue contact with differently-sized pulmonary veins and their ostia.

As shown in **FIG. 4A,** each circumferential flex circuit (CFC) band 34 has a respective radius that is different from the radii of other bands 34. In the illustrated embodiment, the respective radii are as follows:

$$R1 < R2 < R3 < R4$$

Where:

    R1 is the radius of CFC band 34a
    R2 is the radius of CFC band 34b
    R3 is the radius of CFC band 34c
    R4 is the radius of CFC band 34d

In the illustrated embodiment of **FIG. 4A,** the radius of each CFC band decreases as the position of each CFC band increases distally. As illustrated in **FIG. 4A,** the most distal CFC band 34a with tubular region conforming radius R1 is in tissue contact with a smaller ostium OS1, whereas the second most distal CFC band 34b is not in contact with the ostium OS1. In contrast, in **FIG. 4B,** the second most distal band 34b with tubular region conforming radius R2 is in tissue contact with a larger ostium OS2, whereas the most distal CFC band 34A is not in contact with the ostium OS2. Accordingly, the balloon member 30 can be used to ablate pulmonary veins and ostia of different sizes as purposeful placement of the balloon member 30 relative to tissue, including depth of penetration of the tubular region by the conical distal portion 32, ensures complete circumferential contact between tissue and the appropriate CFC band(s).

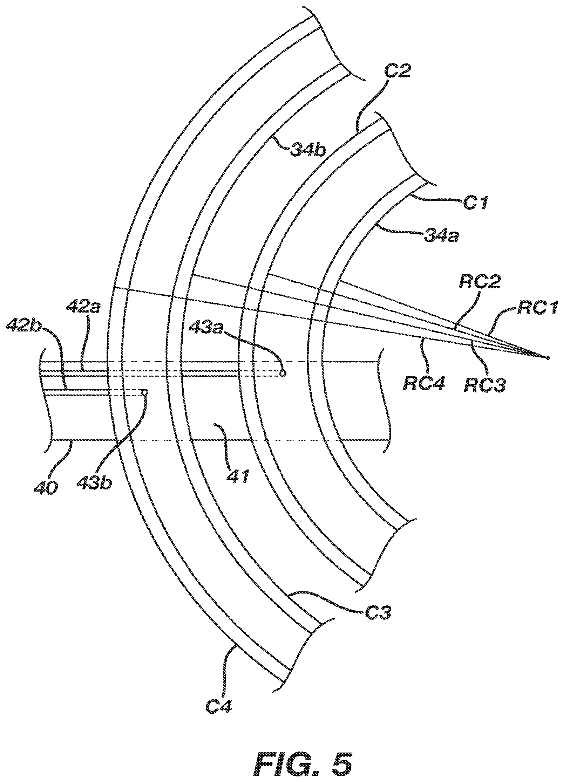

In some embodiments, to construct a CFC band 34 that is conforming to outer surface 33 of the conical distal portion 32, each CFC band (where only bands 34a and 34b are shown in **FIG. 5)** is formed from a flexible planar sheet with curved sides along its length, with more distal sides defined by curvatures of increasingly smaller radii, such that:

$$RC1 < RC2 < RC3 < RC4$$

where:

RC1 is the radius of curvature C1
RC2 is the radius of curvature C2
RC3 is the radius of curvature C3
RC4 is the radius of curvature C4

As understood by one skilled in the art, in some embodiments, the system 10 is configured for bipolar ablation using selected pairs of CFC bands, with one as an active electrode and the other as a return electrode. In other embodiments, the system 10 is configured for unipolar ablation using any one selected band as an active electrode and a separate electrode as a return electrode. Such return electrode may be an external skin patch electrode affixed to the patient's skin or an electrode on a second catheter positioned in another location in the patient's heart.

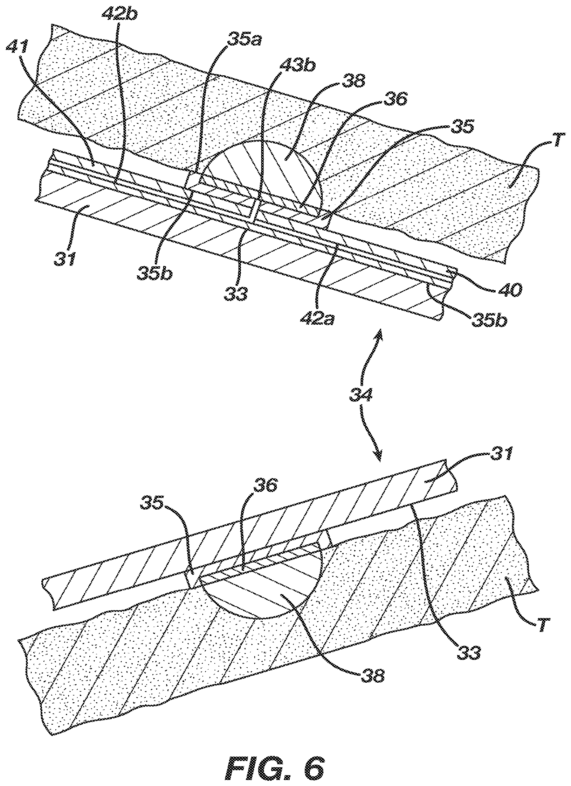

In some embodiments, as shown in **FIG. 6,** the band 34 includes a bio-compatible substrate 35 that is affixed to the membrane 31 of the balloon member 30 without adversely restricting the ability of the balloon member 30 to adopt and transition between the expanded configuration **(FIG. 2A)** and the collapsed configuration **(FIG. 3).** An example of a suitable substrate is KAPTON®, a polyimide tape constructed from a polyimide film that is coated with high-temperature-resistant silicone adhesive, with significant thermal and electrical insulation properties. The substrate 35 has an outer surface 35a (tissue-facing) and an inner surface 35b (non-tissue-facing).

In some embodiments, each CFC band 34 includes a conductive trace 36 disposed on the outer surface 35a of the substrate 35, to function as a singular circumferential electrode wrapped around the outer surface 33 of balloon member 30. Thus, a CFC band 34 that is in contact with tissue in a tubular region T can be selectively energized to create a singular circumferential lesion 38 in tissue that is in contact with the CFC band 34.

As shown in **FIG. 2A, FIG. 4A, FIG. 5** and **FIG. 6,** the ablation balloon member 30 includes a longitudinal flex (LF) circuit 40 to provide electrical connection for each CFC band to wiring through the shaft 25 and into the control handle 12 for coupling to the first driver module 24 of the console 20. The LC circuit 40 has a substrate strip 41 and a dedicated trace 42 for each CFC band 34, for example, 42a, 42b, 42c, 42d. The substrate strip 41 is affixed to the outer surface 33 of the balloon membrane 31 in the longitudinal direction transverse to each of the CFC bands 34. In some embodiments, as shown in **FIG. 6,** the LF circuit 40 may be affixed to the outer surface 33 of the balloon membrane 31 below each CFC band 34, with each dedicated trace 42 connected to a respective circumferential electrode 36 at a respective contact 43, for example, 43a, 43b, 43c, 43d. Alternatively, the LF circuit 40 may be affixed to an inner surface of the balloon membrane, indirectly below each CFC band 34, with each electrical trace formed on the inner surface of the LC circuit and each dedicated trace connected to a respective circumferential electrode at a respective contact. Vias are electrically conductive formations that pass through the electrode and the substrate to electrically couple the electrode with a trace. Blind vias and full vias may be employed as needed for electrical connections and coupling between the substrates and traces. Suitable blind and full vias are described in U.S. Patent No. 10,653,480, the entire contents of which are hereby incorporated by reference.

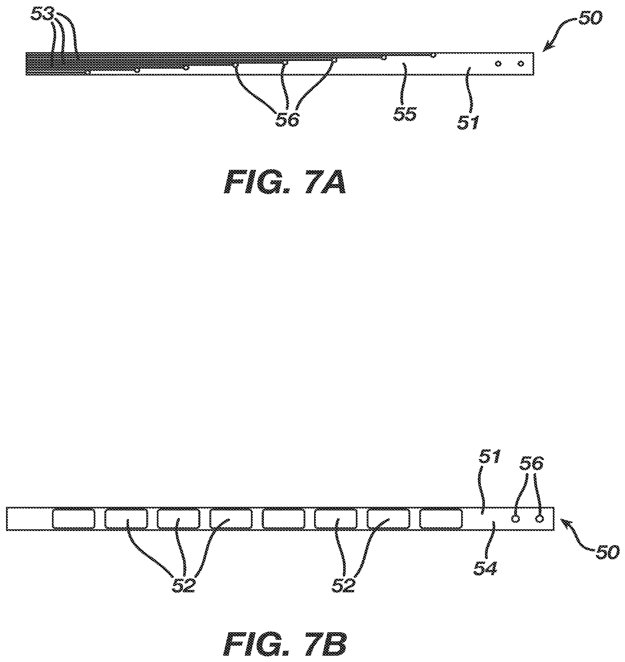

In alternate embodiments, as shown in **FIG. 7A** and **FIG. 7B,** each circumferential flexible circuit (CFC) band 50 includes a substrate 51, a plurality of discrete electrodes (or electrically-conductive members) 52, electrical traces 53 and contacts 54. The electrodes 52 occupy outer or tissue-facing surface 54, spanning at least the longitudinal axis so as to extend circumferentially around the balloon member 30. The electrical traces 53 may be formed on inner (nontissue-facing) surface 55 with each electrode 52 being connected to a respective electrical trace 53 at a respective contact 56. Each contact 56 may comprise a plated conductive via (not shown) that passes through the substrate 51 to the tissue-facing surface 54 and connects a respective trace 53 to a respective electrode 52. The electrodes, contacts and traces may be arranged in other configurations, as desired or appropriate.

It is understood that the traces are connected to suitable wiring that runs through the catheter, for exchanging signals between the console 20 and the electrodes 52. For example, the electrodes 52 may be used for sensing electropotential (EP) signals in the heart and for delivering electrical energy from the first driver module 24 to conduct PFA on tissue in contact with the electrodes.

In some embodiments, the electrodes 52 of the CFC bands 50 may also be used to measure impedance, for example, to determine or infer contact between the electrodes and heart tissue, based on the principle that impedance measured across tissue is greater than impedance measured across bodily fluids such as blood. A low current or voltage prior to ablation is applied by the first driver module 24, acting as a multimeter, across any two selected electrodes 52 of one band 50, or of two different bands 50, and the voltage across these electrodes is processed and measured by the processor of the console 20. When the voltage measured is higher or at or higher than a threshold, electrode in tissue contact is implicated and the system 10 can infer that the electrodes are in contact with tissue as the impedance is presumably being measured across tissue. When the measured voltage is lower than the threshold, the system 10 can infer that the electrodes are not in contact with tissue as the impedance is presumably being measured across blood.

It is understood that while the description herein is directed to circumferential flexible circuit bands 34, the electrodes and the traces in other embodiments may be constructed via vapor deposition onto the membrane 31 of the balloon member 30.

Referring to the illustrated embodiments of **FIG. 2A** and **FIG. 4A,** the configuration of a proximal portion 37 of the balloon member 30 is of lesser consequence relative to the distal portion 32 as the proximal portion 37 is not intended for tissue contact. However, it is understood that the proximal portion 37 should not impede transition of the balloon member 30 between the collapsed configuration and the expanded configuration and ability of the balloon member to be navigated and maneuvered in the patient's body. As better shown in the embodiment of **FIG. 4A,** the proximal portion 37 has a different taper from the taper of the distal portion 32. In some embodiments, whereas the taper (rate at which the radius changes) of the conical distal portion 32 is linear, the taper of the proximal portion 37 between the maximum radius Rmax and the proximal end 30P of the balloon member 30 is nonlinear. In some embodiments, the distal portion 32 has a more gradual taper and the proximal portion 37 has a less gradual taper. In the illustrated embodiment, the balloon member resembles an acorn or a strawberry which shape is also known as conic and can range between a globose conic, a long conic or a necked conic.

In some embodiments, as described above, the balloon member 30 transitions from the collapsed configuration to the expanded configuration either wholly or in part by the introduction of irrigation fluid into the interior of the balloon member 30. The irrigation fluid may be delivered by an irrigation tubing 45 **(FIG. 3)** that is in communication with the fluid conduit 15 that is connected to the fluid source 13 **(FIG. 1).** The irrigation tubing 45 extends through the shaft 25, with a distal end 45D that is coterminous with the distal end 25D of the shaft 25. The irrigation fluid pass from the fluid source 13 may be used to cool surrounding tissue undergoing ablation and thus the irrigation fluid can reach the surrounding tissue by exiting the interior of the balloon member 30 via irrigation apertures 46 formed through the CFC bands 34 and the balloon membrane 31. Additional irrigation apertures 48 may also be formed in portions of the balloon membrane devoid of the CFC bands, as desired or appropriate. In some embodiments, inflation of the balloon member 30 occurs when the irrigation fluid flow rate into the balloon member 30 exceeds the flow rate out of the irrigation apertures 46, 48, and deflation and ultimately collapse of the balloon member occurs when the irrigation fluid flow rate into the balloon member 30 is less than the flow rate out of the irrigation apertures 46, 48.

III. Additional Features of Expandable Assembly

In some embodiments, the balloon member 30 includes sensors disposed on the balloon membrane 31 or on the CFC bands 34. Such sensors may include, for example, pressure sensors, position sensors and/or contact sensors that indicate tissue contact or a proximity between the sensor and the tissue. In some embodiments, as shown in **FIG. 2A** and insert **FIG. 2B,** multiple contact sensors are configured as impedance sensors 58 in lieu of or in addition to the electrodes 52 functioning as impedance sensor, as described herein above. Again, based on the principle that impedance measured across tissue is greater than impedance measured across bodily fluids such as blood, an impedance sensor 58 includes an electrode 59 configured for tissue contact. A low current or voltage prior to ablation is applied by the first driver module 24, acting as a multimeter, across an electrode 59 and an external skin patch electrode 60 on the patient's body and the voltage across the the electrode 59 and the patch electrode 60 is measured. When the voltage measured is higher or at or above a threshold, the sensor 58 implicates tissue contact and the system 10 can predict or infer that the electrode 59 is in contact with tissue as the impedance is presumably measured across tissue. When the measured voltage is lower or below a threshold, the system 10 can predict or infer that the electrode 59 is not in contact with tissue as the impedance is presumably measured across blood. Impedance may be measured at a CFC band 34 in detecting contact of the CFC band with tissue where the electrode 59 is disposed at a location at or near a CFC band 34. As shown in **FIG. 2A and FIG. 2B,** the electrode 59 is located at a location within a CFC band 34, surrounded entirely circumferentially with an exclusion zone 62 filled with an electrically insulating material to physically and electrically isolate the electrode 59 from the CFC band 34. The electrode 59 may be electrically connected by a via to a dedicated trace in the CFC band or wiring extending below the balloon membrane. Suitable blind and full vias are described in U.S. Patent No. 10,653,480, the entire contents of which are hereby incorporated by reference.

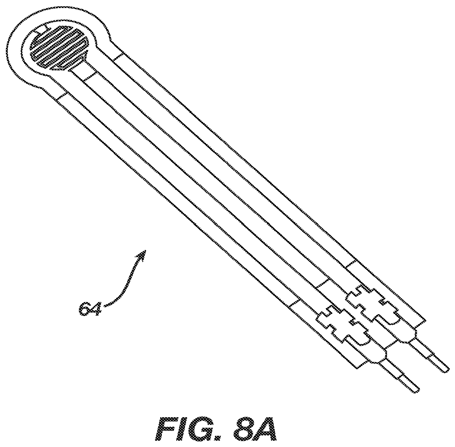

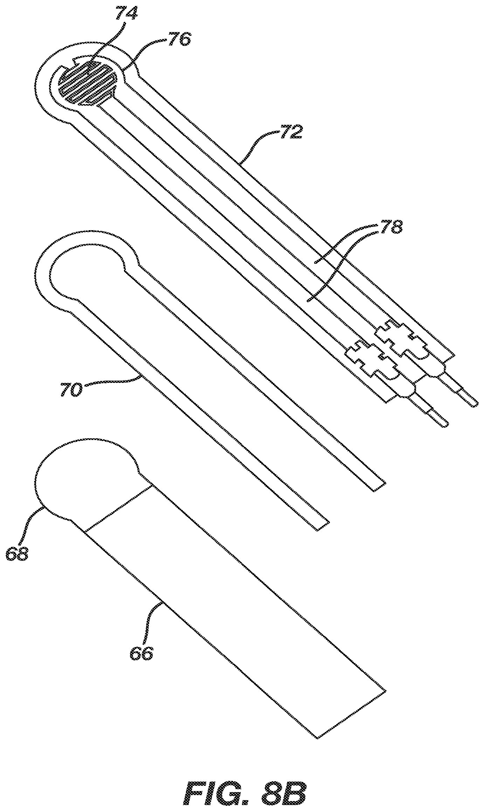

Contact sensors may include force sensitive resistors (FSR), as shown in **FIG. 8A.** FSRs 64, also known as touch sensors or piezoresistive force sensors, measure a force that is applied to them by varying its resistance in response to the pressure applied to a sensing area. The greater the pressure, the lower the resistance. The signals generated by the FSRs 64 can be calibrated to determine when tissue contact occurs. The system 10 can also predict tissue contact in response to signals from the FSRs 64. As shown in **FIG. 8B,** the FSR 64 includes a first layer 66 of flexible substrate with an area of printed semi-conductor 68, a layer of adhesive 70, and a second layer 72 of flexible substrate with printed interdigitated electrodes (IDE) 74 as an active area 76 and conducting tails 78 which are connected to wiring or traces through which the first driver module 24 can apply a current and measure the resistance as a measure of pressure or contact. In some embodiments, the balloon membrane 30 include a plurality of FSRs 64 affixed to the outer surface 33 of the balloon membrane 30 to detect pressure as an indication of contact with tissue of the tubular region.

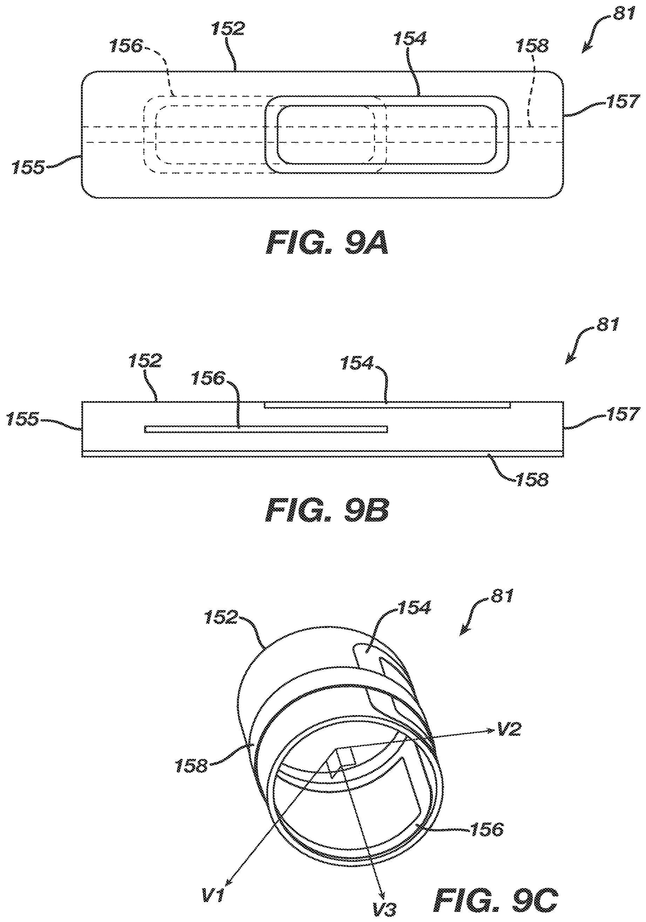

In some embodiments, position sensors are configured as a flexible substrate with sensing coils embedded in layers of the substrate. As described herein, the substrate may be a bio-compatible material, e.g., KAPTON® and the coil is constructed of an electrically conductive material. The sensing coils are responsive to the external magnetic field generators 17 underneath the patient's bed in generating signals indicative of the location and position of the coils in the patient's body. The position sensing flexible substrate may have one coil as a single-axis position sensor, two coils as a dual-axes sensor or three coils as a triple-axes sensor. As shown in **FIG. 9A,** a triple-axes sensor (TAS) 81 has a substrate 152 formed of any suitable material, e.g., KAPTON® and three embedded coils 154, 156, 158 formed on and within layers of the substrate. The substrate has an elongated form, with a longitudinal axis LA and opposing ends 155, 157. The coils are made from a conductive material, e.g., copper. In some embodiments, the coil 154 is formed on an upper surface of the substrate 152, the coil 156 is embedded within an internal layer of the substrate 152, and the coil 158 is formed on a lower surface of the substrate 152. In this arrangement, the coils are electrically isolated from each other by suitable dielectric layers. Each of the coils 154, 156 has a substantially symmetrical shape, e.g., a rectangular close-loop shape whereas the coil 158 comprises a stripe that passes end-to-end along the substrate 152. Each of the coils is electrically connected to the catheter 28 via electrical circuit traces (not shown) printed on the substrate. In **FIG. 9C,** the substrate 152 is rolled into a cylindrical shape with ends 155, 157 being joined, where axis V1 of coil 154, axis V2 of coil 156 and axis V3 of coil 158 are substantially orthogonal to each other. The coils 154, 156 are formed with a certain offset relative to each other on the substrate 152. The offset is calculated so that after the substrate is rolled, the two coils are oriented with mutually-orthogonal axes.

As shown in the embodiment of **FIG. 2A,** the balloon member includes a first TAS 81 affixed to the distal end 25D of the shaft 25 and a second TAS 80 affixed to the balloon membrane 31 near a distal end of the balloon member 30, each with coils 154, 156, 158 configured to provide signals indicative of position in response to the external magnetic field generators 17.

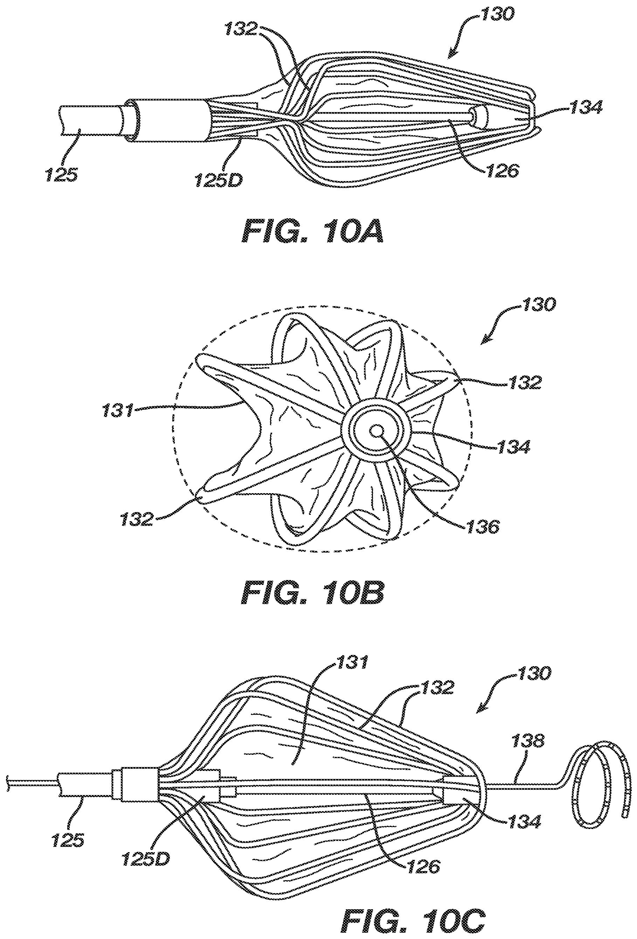

In other embodiments, as shown in **FIG. 10A,** an ablation catheter with balloon member 130 includes a framework comprising a plurality of elongated elastically-flexible splines 132 that extend longitudinally with distal ends that converge to form a distal hub 134 of the balloon member 130 and proximal ends that are mounted in a distal end 125D of an elongated deflectable shaft 125. A center shaft or pusher 126 that extends through a lumen of the shaft 125 and is longitudinally movable relative to the shaft 125 extends longitudinally through the balloon member 130, from the distal end 125D of the shaft 125 to the distal hub 134. The flexible splines 132 are configured to bow radially in their neutral state to assume an expanded configuration **(FIG. 10C)** but can be resiliently folded **(FIG. 10B)** and compressed into a collapsed configuration when constrained, e.g., by an outer sheath 129 **(FIG. 10C).** A distal end of the pusher 126 is fixed to the distal hub 134 such that advancement and withdrawal of the pusher as manipulated at a proximal end of the pusher by the physician proximal of the control handle 12 **(FIG. 1)** collapses and expands the balloon member 130, respectively. In some embodiments, an irrigation tubing 127 extends through the lumen of the shaft 125 and has a distal end 127D that terminates in the interior of the balloon member 130 to pass irrigation fluid into the balloon member 130.

In some embodiments, the pusher 126 has a lumen that communicates and is aligned with an opening 136 **(FIG. 10B)** in the distal hub 134. The lumen and the opening 136 are sized to accommodate another instrument to pass through. Accordingly, the ablation catheter can be used with another catheter, e.g., a lasso catheter 138 **(FIG. 10C),** that is inserted through the lumen of the pusher 126 and passes through the opening 136 to be advanced distal of the balloon member 130.

IV. Example of Method for Use with Expandable Assembly with Circumferential Flex Circuit Bands

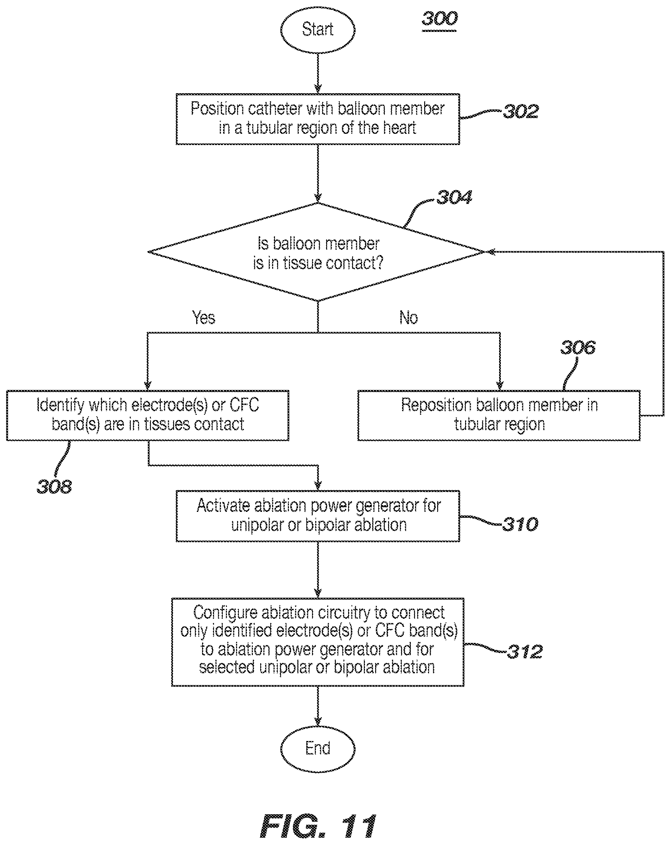

**FIG. 11** is a flowchart illustrating an embodiment of a method 300 using an electrophysiology system to ablate a tubular region of the heart using an expandable catheter having multiple electrode bands of different circumferential sizes which can be selectively energized depending on detected tissue contact to create a full circumferential lesion via unipolar or bipolar ablation in treating heart arrythmia. In accordance with an embodiment of the present invention. At Block 302, method 300 includes positioning the catheter with the balloon member in a tubular region of the heart. The second driver module drives the external magnetic field generators. The single axis position sensors on the balloon member and/or the tri-axes position sensor at the distal end of the shaft in response to the external magnetic field generators generate signals processed by the first driver module to determine position of the balloon member, which may be displayed on the display superimposed on a 3-D anatomical image of the tubular region. The physician can deflect the catheter using control handle to actuate a deflection section of the shaft in maneuvering the balloon member into the tubular region. Depending on the size of the tubular region relative to the balloon member, the balloon member can extend into the tubular region at different depths. In some embodiments, pushability of the shaft provides the physician with tactile sensory as to when the balloon member has been sufficiently advanced in the tubular region to reach a maximum depth.

At Query 304, the method includes determining whether the balloon member is in tissue contact with the heart, which may include measurements of impedance via the CFC bands 34 or the contact impedance sensor, as described hereinabove. Prior to ablation, the first driver module sends an electrical current to a circuit that includes CFC bands or the electrodes of the impedance sensor as the active electrode and other CFC bands or body surface patch electrodes as the return electrode to create a voltage therebetween that is used to measure the impedance and compares the impedance to a threshold impedance or calibrated impedance to determine whether tissue contact can be inferred. The first driver module can send electrical current to selected CFC bands and contact impedance sensors or to all such electrical conductors.

At Block 306, if tissue contact has not been determined, the physician can reposition the balloon member, for example, by withdrawing and reinserting the balloon member, increasing and/or decreasing the expansion of the balloon member, and/or by rotating the balloon member about is longitudinal axis. Returning to Block 304, tissue contact is again assessed. This loop between Block 304 and Block 306 may continue as needed until tissue contact (or sufficient tissue contact) has been determined. It is understood that the physician may continue readjusting the position of the balloon member until electrode(s) or a CFC band is in complete circumferential contact with tissue of the tubular region for creating a complete circumferential lesion.

At Block 308, the processor identifies any and all electrode(s) or CFC band(s) identified as having tissue contact or sufficient tissue contact. The processor may also drive the display to display information on the locations and/or the identified electrodes a or CFC band(s) on the balloon member indicated to be in tissue contact.

At Block 310, the physician activates the first driver module to send ablation energy to the electrode(s) or CFC band(s) identified to be in complete circumferential tissue contact. The physician may also select unipolar or bipolar ablation via controls in the console.

At Block 312, the processor configures the ablation circuitry in the EP system to connect ablation power generator to solely the electrode(s) or CFC band(s) indicated to be in complete circumferential tissue contact. The ablation circuitry may include a switch circuitry configured to be responsive to the processor in completing electrical connections between the ablation power generator and the identified electrode(s) or CFC band(s) in either unipolar or bipolar ablation in forming a complete circumferential lesion in the tubular region. Selective electrical connection of electrodes reduces the exposure of the patient to excess ablation energy. It is understood that the ablation electrodes or the CFC bands themselves may be configured to function as the impedance sensors in lieu of or in addition to contact impedance electrodes, as described hereinabove.

V. Examples of Combinations

The following examples relate to various non-exhaustive ways in which the teachings herein may be combined or applied. The following examples are not intended to restrict the coverage of any claims that may be presented at any time in this application or in subsequent filings of this application. No disclaimer is intended. The following examples are being provided for nothing more than merely illustrative purposes. It is contemplated that the various teachings herein may be arranged and applied in numerous other ways. It is also contemplated that some variations may omit certain features referred to in the below examples. Therefore, none of the aspects or features referred to below should be deemed critical unless otherwise explicitly indicated as such at a later date by the inventors or by a successor in interest to the inventors. If any claims are presented in this application or in subsequent filings related to this application that include additional features beyond those referred to below, those additional features shall not be presumed to have been added for any reason relating to patentability.

Examples described herein are directed to an electrophysiology system using a catheter with an expandable end effector configured with multiple concentric bands of circumferential ablation flexible circuits, each configured with a different circumference at a different location along a longitudinal axis of the end effector.

**Example 1**

A catheter system comprising: (a) a catheter including an ablation balloon member with a membrane, and a plurality of ablation flexible circuits disposed on the membrane, the ablation balloon member defining a longitudinal axis and each ablation flexible circuit extending circumferentially around the longitudinal axis with a different respective circumference at a different respective location along the longitudinal axis; (b) a contact sensor disposed on the membrane and configured to provide contact signals indicating contact between the balloon member and tissue of a tubular region; (c) a generator configured to provide electrical current; and a controller configured to selectively transmit the electrical current to a selected flexible circuit in response to the contact signals.

Example 2

The catheter system of Example 1, wherein the contact signals indicate contact of the selected ablation flexible circuit with the tissue of the tubular region.

Example 3

The catheter system of Example 1 or 2, wherein each ablation flexible circuit includes a substrate and an electrically-conductive trace, the substrate configured as an elongated strip configured with a length and a width, and then electrically-conductive trace extends along the length of the strip.

Example 4

The catheter system of Example 3, wherein the electrically-conductive trace of each ablation flexible circuit is configured to receive the electrical current for ablating tissue.

Example 5

The catheter system of Example 3, wherein a first electrically-conductive trace is configured as an active electrode and a second electrically-conductive trace is configured as a return electrode, the active and return electrodes configured to define a circuit in a patient's body in response to the electrical current, the circuit having voltage which is determinative of an impedance from tissue contact of the first electrically-conductive trace is inferred.

Example 6

The catheter system of Example 3, wherein a first electrically-conductive trace is configured as an active electrode and a surface patch electrode is configured as a return electrode, the active and return electrodes configured to define a circuit in a patient's body in response to the electrical current, the circuit having voltage which is determinative of an impedance from which of tissue contact of the first electrically-conductive trace is inferred.

Example 7

The catheter of any of the foregoing Examples, wherein the contact sensor includes an impedance sensor.

Example 8

The catheter of any of the foregoing Examples, wherein the contact sensor includes a force sensitive resistor.

Example 9

The catheter of any of the foregoing Examples, wherein the balloon member is configured to transition between an expanded configuration and a collapsed configuration.

Example 10

The catheter of Example 9 wherein the balloon member in the expanded configuration has a conic configuration defined by a maximum circumference.

Example 11

The catheter of Example 10, wherein the plurality of flexible circuits are disposed on the balloon member distal of the maximum circumference.

Example 12

The catheter of Example 10, wherein the balloon member has a distal portion distal of the maximum circumference and a proximal portion of the maximum circumference, the distal portion configured with a linear taper and the proximal portion configured with a nonlinear taper.

Example 13

The catheter of Example 10, wherein the conic configuration includes a globose conic, a long conic or a necked conic.

Example 14

An ablation catheter, comprising: (a) a control handle; (b) an elongated shaft distal of the control handle; and (c)an ablation end effector including:(i) a balloon member defining a longitudinal axis between a proximal end and a distal end, the balloon member configured to transition between a collapsed configuration and an expanded configuration; (ii) a plurality of flexible circuit bands disposed on the balloon member, each flexible circuit band extending circumferentially around the longitudinal axis with a different respective radius at a different respective location along the longitudinal axis, each flexible circuit band including a circumferential electrode configured to contact a circumferential surface of tissue in a tubular region of the heart; and (iii) a sensor configured to generate signals indicative of a proximity between tissue and a circumferential electrode.

Example 15

The ablation catheter of Example 14, wherein the sensor includes an impedance sensor.

Example 16

The ablation catheter of Example 14, wherein the sensor includes a force sensitive resistor.

Example 17

The ablation catheter of any of Examples 14-16, further comprising a longitudinal flexible circuit with a plurality of traces, each trace electrically coupled to a respective circumferential electrode.

Example 18

The ablation catheter of any of Examples 14-17, further comprising a position sensor.

Example 19

The ablation catheter of Example 18, wherein the position sensor includes a tri-axes sensor.

Example 20

The ablation catheter of Example 18, wherein the tri-axes position sensor is mounted at or near a distal end of the elongated shaft.

Example 21

The ablation catheter of Example 19 or 20, wherein the tri-axes position sensor is disposed on the balloon member.

Example 22

The ablation catheter of any of Examples 14-21, wherein the balloon member includes: (a) a framework of flexible spines, each spline configured with a distal end and a proximal end, the distal ends of the splines converging at the distal end of the balloon member, the splines configured to bow out radially from the longitudinal axis in supporting the balloon member in the expanded configuration; and (b) a second elongated shaft extending through the elongated shaft, a distal end of the second shaft being coupled to the distal end of the balloon member such that longitudinal movement of the second shaft in a distal direction relative to the elongated shaft transitions the balloon member from the expanded configuration to the collapsed configuration.

Example 23

The ablation catheter of any of Examples 14-22, further comprising an irrigation tubing extending through the elongated shaft and configured with a distal end terminating in an interior of the balloon member.

Example 24

A catheter system comprising: (a) a catheter including: (i) an ablation balloon member with a membrane, the balloon member configured with a conical shape with a distal portion and a proximal portion delineated by a maximum radius Rmax, the distal portion configured with a first taper and the proximal portion configured with a second taper; (ii) a plurality of ablation flexible circuits disposed on the membrane in the distal portion of the balloon member, the ablation balloon member defining a longitudinal axis and each ablation flexible circuit extending circumferentially around the longitudinal axis with a different respective radius at a different respective location along the longitudinal axis; and (iii) a contact sensor disposed on the membrane and configured to provide signals implicating contact between the balloon member and tissue of a tubular region; (b) a generator configured to provide pulsed field ablation energy; and (c) a controller configured to selectively transmit the pulsed field ablation energy to a selected flexible circuit in response to the signals.

Example 25

The catheter system of Example 24, wherein the controller is configured to selectively transmit the pulsed field ablation energy to a selected flexible circuit as an active electrode in a bipolar configuration.

Example 26

The catheter system of Example 24, wherein the controller is configured to selectively transmit the pulsed field ablation energy to a selected flexible circuit as an active electrode in a unipolar configuration.

Example 27

The catheter system of Example 24, wherein the generator is also configured to provide an electrical current, a first ablation flexible circuit is configured as an active electrode and a second ablation flexible circuit is configured as a return electrode, the active and return electrodes configured to define a circuit in a patient's body in response to the electrical current, the circuit having a voltage which is determinative of an impedance from which tissue contact of the first ablation flexible circuit is inferred.

Example 28

The catheter system of Example 24, wherein the energy generator is also configured to provide an electrical current, a first ablation flexible circuit is configured as an active electrode and a body surface patch electrode is configured as a return electrode, the active and return electrodes configured to define a circuit in a patient's body in response to the electrical current, the circuit having a voltage which is determinative of an impedance from which tissue contact of the first ablation flexible circuit is inferred.

Example 29

The catheter system of any of Examples 24 - 26, wherein the contact sensor includes an impedance sensor.

Example 30

The catheter of Example 29, wherein the impedance sensor generates signals and the controller is configured to determine contact between the impedance sensor and the tissue based on the signals.

Example 31

The catheter of Example 29, wherein the impedance sensor generates signals and the controller is configured to determine a position of the impedance sensor in a patient's body.

Example 32

The catheter of Example 29, wherein the impedance sensor includes an impedance electrode disposed on the membrane and surrounded by an ablation flexible circuit, the impedance electrode physically and electrically isolated from the ablation flexible circuit by an exclusion zone.

Example 33

The catheter system of Example 24, wherein the contact sensor includes a force sensitive resistor.

Example 34

The catheter system of Example 24, wherein the catheter includes a tri-axis position sensor comprising a flexible substrate and three trace coils arranged orthogonally to each other.

Example 35

The catheter system of any of Examples 24 - 34, wherein the balloon member in the expanded configuration has a conic configuration defined by a maximum radius and the plurality of flexible circuits are disposed on the balloon member distal of the maximum radius.

VI. Miscellaneous

Any of the versions of the instruments described herein may include various other features in addition to or in lieu of those described above. While the examples herein are described mainly in the context of electrosurgical instruments, various teachings herein may be readily applied to a variety of other types of devices. By way of example only, the various teachings herein may be readily applied to other types of electrosurgical instruments, tissue graspers, tissue retrieval pouch deploying instruments, surgical staplers, surgical clip appliers, ultrasonic surgical instruments, etc. The teachings herein may be readily applied to any of the instruments described in any of the references cited herein. Any of the devices herein may also include one or more of the various features disclosed in any of the various references that are incorporated by reference herein. The teachings herein may thus be readily combined with the teachings of any of the references cited herein in numerous ways. Other types of instruments into which the teachings herein may be incorporated will be apparent to those skilled in the art in view of the teachings herein.

It should be appreciated that any patent, publication, or other disclosure material, in whole or in part, that is said to be incorporated by reference herein is incorporated herein only to the extent that the incorporated material does not conflict with existing definitions or other disclosure material set forth in this disclosure. The disclosure as explicitly set forth herein supersedes any conflicting material incorporated herein by reference. Any material, or portion thereof, that is said to be incorporated by reference herein, but which conflicts with existing definitions or other disclosure material set forth herein will only be incorporated to the extent that no conflict arises between that incorporated material and the existing disclosure.

The terms "proximal" and "distal" are defined herein relative to a surgeon, robotic arm, or other operator or structure grasping a surgical instrument having a distal surgical end effector. The term "proximal" refers the position of an element closer to the surgeon, robotic arm, or other operator or structure; and the term "distal" refers to the position of an element closer to the surgical end effector of the surgical instrument and further away from the surgeon or other operator or structure.

As used herein, an element or step recited in the singular and proceeded with the word "a" or "an" should be understood as not excluding plural of said elements or steps, unless such exclusion is explicitly stated. Furthermore, references to "one example" or "an example" are not intended to be interpreted as excluding the existence of additional examples that also incorporate the recited features. The use of "including," "comprising," or "having" and variations thereof herein is meant to encompass the items listed thereafter, and equivalents thereof, as well as additional items.

As used herein in the specification and claims, including as used in the examples and unless otherwise expressly specified, the terms "about" or "approximately" for any numerical values or ranges indicate a suitable dimensional tolerance, or other form of reasonable expected range, that allows the part or collection of components to function for its intended purpose as described herein. More specifically, "about" or "approximately" may refer to the range of values that are within ±10% of the recited value (e.g., "about 100" may refer to the range of values from 90 to 110, including 90, 110, 100, and all other values within the range of 90 and 110). Any numerical values given herein should also be understood to include about or approximately that value unless the context indicates otherwise. For example, if the value "10" is disclosed, then "about 10" is also disclosed. Any numerical range recited herein is intended to include all subranges subsumed therein. The terms "approximately" and "about" are thus utilized herein to represent the degree by which a quantitative representation may vary from a stated reference without resulting in a change in the basic function of the subject matter at issue.

The term "substantially" is also utilized herein to represent the degree by which a quantitative representation may vary from a stated reference without resulting in a change in the basic function of the subject matter at issue. The term "substantially" shall therefore be understood to include a range of conditions or results that provide a functional equivalent to an explicitly stated condition or result. For instance, if a task is "substantially complete," the result of the task having been substantially completed is functionally equivalent to the result that would have been achieved if the task had been perfectly completed. As another non-limiting example, a component that is "substantially straight" or "substantially flat," an apparatus including a component that is "substantially straight" or "substantially flat" may provide a result or effect that is functionally equivalent to a result or effect that would be achieved by the same apparatus including the same component in a perfectly straight or perfectly flat configuration. The range implied by the term "substantially" should also be read to include the perfect result that is within that range. Thus, the term "substantially complete" shall be read as including "perfectly complete" while also including a range of completeness that is functionally equivalent to perfectly complete. As another example, terms such as "substantially straight" and "substantially flat" shall be read as including "perfectly straight" and "perfectly flat," respectively; while also including a range of straightness or flatness that is functionally equivalent to perfectly straight or flat, respectively. As with the terms "approximately" and "about," the term "substantially" may indicate a suitable dimensional tolerance, or other form of reasonable expected range, that allows a part or collection of components to function for its intended purpose as described herein.

The limitations of the following claims are not written in means-plus-function format and are not intended to be interpreted based on 35 U.S.C. §112(f), unless such claim limitations expressly use the phrase "means for" followed by a statement of function void of further structure.

Versions described above may be designed to be disposed of after a single use, or they can be designed to be used multiple times. Versions may, in either or both cases, be reconditioned for reuse after at least one use. Reconditioning may include any combination of the steps of disassembly of the device, followed by cleaning or replacement of particular pieces, and subsequent reassembly. In particular, some versions of the device may be disassembled, and any number of the particular pieces or parts of the device may be selectively replaced or removed in any combination. Upon cleaning and/or replacement of particular parts, some versions of the device may be reassembled for subsequent use either at a reconditioning facility, or by an operator immediately prior to a procedure. Those skilled in the art will appreciate that reconditioning of a device may utilize a variety of techniques for disassembly, cleaning/replacement, and reassembly. Use of such techniques, and the resulting reconditioned device, are all within the scope of the present application.

By way of example only, versions described herein may be sterilized before and/or after a procedure. In one sterilization technique, the device is placed in a closed and sealed container, such as a plastic or TYVEK bag. The container and device may then be placed in a field of radiation that can penetrate the container, such as gamma radiation, x-rays, or high-energy electrons. The radiation may kill bacteria on the device and in the container. The sterilized device may then be stored in the sterile container for later use. A device may also be sterilized using any other technique known in the art, including but not limited to beta or gamma radiation, ethylene oxide, or steam.

The preceding description has been presented with reference to presently preferred embodiments of the invention. Workers skilled in the art and technology to which this invention pertains will appreciate that alterations and changes in the described structure may be practiced without meaningfully departing from the principal, spirit and scope of this invention. Any feature or structure disclosed in one embodiment may be incorporated in lieu of or in addition to other features of any other embodiments, as needed or appropriate. As understood by one of ordinary skill in the art, the drawings are not necessarily to scale. Accordingly, the foregoing description should not be read as pertaining only to the precise structures described and illustrated in the accompanying drawings, but rather should be read consistent with and as support to the following claims which are to have their fullest and fair scope.

It is noted that the electrodes described and illustrated herein are not limited to mapping (i.e., sensing signals or recording signals) but can be used to deliver energy such as RF (alternating cycle) or IRE (DC pulses) in bipolar or unipolar mode alone or in combination with the mapping or sensing function. In the application for IRE, and by way of example only, the electrodes may be configured to deliver at least 900V per electrode with a current of at least 20 amperes over a number of pulses sufficient to cause cell apoptosis. It is also noted that the mapping and ablation described herein may be applied to other tissue and organs beyond the heart.

It should be understood that any of the embodiments described herein may include various other features in addition to or in lieu of those described above. By way of example only, any of the embodiments described herein may also include one or more of the various features disclosed in any of the various references that are incorporated by reference herein.

It should be understood that any one or more of the teachings, expressions, embodiments, examples, etc. described herein may be combined with any one or more of the other teachings, expressions, embodiments, examples, etc. that are described herein. The above-described teachings, expressions, embodiments, examples, etc. should therefore not be viewed in isolation relative to each other. Various suitable ways in which the teachings herein may be combined will be readily apparent to those skilled in the art in view of the teachings herein. Such modifications and variations are intended to be included within the scope of the claims.

It should be appreciated that any patent, publication, or other disclosure material, in whole or in part, that is said to be incorporated by reference herein is incorporated herein only to the extent that the incorporated material does not conflict with existing definitions, statements, or other disclosure material set forth in this disclosure. As such, and to the extent necessary, the disclosure as explicitly set forth herein supersedes any conflicting material incorporated herein by reference. Any material, or portion thereof, that is said to be incorporated by reference herein, but which conflicts with existing definitions, statements, or other disclosure material set forth herein will only be incorporated to the extent that no conflict arises between that incorporated material and the existing disclosure material.

Having shown and described various versions of the present invention, further adaptations of the methods and systems described herein may be accomplished by appropriate modifications by one skilled in the art without departing from the scope of the present invention. Several of such potential modifications have been mentioned, and others will be apparent to those skilled in the art. For instance, the examples, versions, geometrics, materials, dimensions, ratios, steps, and the like discussed above are illustrative and are not required. Accordingly, the scope of the present invention should be considered in terms of the following claims and is understood not to be limited to the details of structure and operation shown and described in the specification and drawings.

## Claims

1. A catheter system comprising:

a catheter including an ablation balloon member with a membrane, and a plurality of ablation flexible circuits disposed on the membrane, the ablation balloon member defining a longitudinal axis and each ablation flexible circuit extending circumferentially around the longitudinal axis with a different respective circumference at a different respective location along the longitudinal axis;
a contact sensor disposed on the membrane and configured to provide contact signals indicating contact between the balloon member and tissue of a tubular region;
a generator configured to provide electrical current; and
a controller configured to selectively transmit the electrical current to a selected flexible circuit in response to the contact signals.

2. The catheter system of claim 1, wherein the contact signals indicate contact of the selected ablation flexible circuit with the tissue of the tubular region.

3. The catheter system of claim 1, wherein each ablation flexible circuit includes a substrate and an electrically-conductive trace, the substrate configured as an elongated strip with a length and a width, and the electrically-conductive trace extends along the length of the strip.

4. The catheter system of claim 3, wherein the electrically-conductive trace of each ablation flexible circuit is configured to receive the electrical current for ablating tissue.

5. The catheter system of claim 3, wherein i) a first electrically-conductive trace is configured as an active electrode and a second electrically-conductive trace is configured as a return electrode, the active and return electrodes configured to define a circuit in a patient's body in response to the electrical current, the circuit having voltage which is determinative of an impedance from tissue contact of the first electrically-conductive trace can be inferred, or ii) a first electrically-conductive trace is configured as an active electrode and a surface patch electrode is configured as a return electrode, the active and return electrodes configured to define a circuit in a patient's body in response to the electrical current, the circuit having voltage which is determinative of an impedance from which of tissue contact of the first electrically-conductive trace can be inferred.

6. The catheter of claim 1, wherein the contact sensor includes an impedance sensor or a force sensitive resistor.

7. The catheter of claim 1, wherein the balloon member is configured to transition between an expanded configuration and a collapsed configuration.

8. The catheter of claim 7, wherein the balloon member in the expanded configuration has a conic configuration defined by a maximum circumference.

9. The catheter of claim 8, wherein i) the plurality of flexible circuits are disposed on the balloon member distal of the maximum circumference, ii) the balloon member has a distal portion distal of the maximum circumference and a proximal portion of the maximum circumference, the distal portion configured with a linear taper and the proximal portion configured with a nonlinear taper, or ii) the conic configuration includes a globose conic, a long conic or a necked conic.

10. An ablation catheter, comprising:

a control handle;
an elongated shaft distal of the control handle; and
an ablation end effector including:

a balloon member defining a longitudinal axis between a proximal end and a distal end, the balloon member configured to transition between a collapsed configuration and an expanded configuration
a plurality of flexible circuit bands disposed on the balloon member, each flexible circuit band extending circumferentially around the longitudinal axis with a different respective radius at a different respective location along the longitudinal axis, each flexible circuit band including a circumferential electrode configured to contact a circumferential surface of tissue in a tubular region of the heart; and
a sensor configured to generate signals indicative of a proximity between tissue and a circumferential electrode.

11. The ablation catheter of claim 10, wherein the balloon member includes:

a framework of flexible spines, each spline configured with a distal end and a proximal end, the distal ends of the splines converging at the distal end of the balloon member, the splines configured to bow out radially from the longitudinal axis in supporting the balloon member in the expanded configuration; and

a second elongated shaft extending through the elongated shaft, a distal end of the second shaft being coupled to the distal end of the balloon member such that longitudinal movement of the second shaft in a distal direction relative to the elongated shaft transitions the balloon member from the expanded configuration to the collapsed configuration.

12. A catheter system comprising:

a catheter including:

an ablation balloon member with a membrane, the balloon member configured with a conical shape with a distal portion and a proximal portion delineated by a maximum radius Rmax, the distal portion configured with a first taper and the proximal portion configured with a second taper;
a plurality of ablation flexible circuits disposed on the membrane in the distal portion of the balloon member, the ablation balloon member defining a longitudinal axis and each ablation flexible circuit extending circumferentially around the longitudinal axis with a different respective radius at a different respective location along the longitudinal axis; and
a contact sensor disposed on the membrane and configured to provide signals implicating contact between the balloon member and tissue of a tubular region;

an energy generator configured to provide pulsed field ablation energy; and
a controller configured to selectively transmit the pulsed field ablation energy to a selected flexible circuit in response to the signals.

13. The catheter system of claim 12, wherein the controller is configured to selectively transmit the pulsed field ablation energy to a selected flexible circuit as an active electrode in a bipolar configuration or as an active electrode in a unipolar configuration.

14. The catheter system of claim 12, wherein the energy generator is also configured to provide an electrical current, a first ablation flexible circuit is configured as an active electrode and a second ablation flexible circuit is configured as a return electrode, the active and return electrodes configured to define a circuit in a patient's body in response to the electrical current, the circuit having a voltage which is determinative of an impedance from which tissue contact of the first ablation flexible circuit can be inferred.

15. The catheter system of claim 12, wherein the energy generator is also configured to provide an electrical current, a first ablation flexible circuit is configured as an active electrode and a body surface patch electrode is configured as a return electrode, the active and return electrodes configured to define a circuit in a patient's body in response to the electrical current, the circuit having a voltage which is determinative of an impedance from which tissue contact of the first ablation flexible circuit can be inferred.

**FIG. 1**

FIG. 2A

FIG. 2B

*FIG. 3*

**FIG. 4A**

**FIG. 4B**

**FIG. 5**

**FIG. 6**

**FIG. 7A**

**FIG. 7B**

**FIG. 8A**

**FIG. 8B**

**FIG. 9A**

**FIG. 9B**

**FIG. 9C**

**FIG. 10A**

**FIG. 10B**

**FIG. 10C**

FIG. 11

Europäisches
Patentamt

European
Patent Office

Office européen
des brevets

# EUROPEAN SEARCH REPORT

**Application Number**

EP 25 21 2655

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2012/071870 A1 (SALAHIEH AMR [US] ET AL) 22 March 2012 (2012-03-22) | 1-9 | INV.<br>A61B18/14 |
| Y | * abstract; figures 2B, 2C, 5F, 18F *<br>* paragraph [0098] - paragraph [0273] * | 10-15 | |
| Y | US 2023/414274 A1 (MOSS KEVIN L [US] ET AL) 28 December 2023 (2023-12-28)<br>* abstract; figure 17B *<br>* paragraph [0082] - paragraph [0165] * | 10-15 | |
| A | US 2016/192981 A1 (DIMMER STEVEN C [US] ET AL) 7 July 2016 (2016-07-07)<br>* abstract; figure 24 * | 1-15 | |

**TECHNICAL FIELDS SEARCHED (IPC)**

A61B

The present search report has been drawn up for all claims

| Place of search | Date of completion of the search | Examiner |
|---|---|---|
| Munich | 10 March 2026 | Wetzig, Thomas |

CATEGORY OF CITED DOCUMENTS

X : particularly relevant if taken alone
Y : particularly relevant if combined with another
document of the same category
A : technological background
O : non-written disclosure
P : intermediate document

T : theory or principle underlying the invention
E : earlier patent document, but published on, or
after the filing date
D : document cited in the application
L : document cited for other reasons

& : member of the same patent family, corresponding
document

EPO FORM 1503 03.82 (P04C01)

## ANNEX TO THE EUROPEAN SEARCH REPORT
## ON EUROPEAN PATENT APPLICATION NO.

EP 25 21 2655

This annex lists the patent family members relating to the patent documents cited in the above-mentioned European search report.
The members are as contained in the European Patent Office EDP file on
The European Patent Office is in no way liable for these particulars which are merely given for the purpose of information.

10-03-2026

| Patent document cited in search report | Publication date | Patent family member(s) | | Publication date |
|---|---|---|---|---|
| US 2012071870 A1 | 22-03-2012 | AU | 2011252976 A1 | 08-11-2012 |
| | | AU | 2017202014 A1 | 27-04-2017 |
| | | CA | 2797130 A1 | 17-11-2011 |
| | | CN | 103118620 A | 22-05-2013 |
| | | CN | 105105844 A | 02-12-2015 |
| | | EP | 2568905 A2 | 20-03-2013 |
| | | JP | 5792802 B2 | 14-10-2015 |
| | | JP | 2013529109 A | 18-07-2013 |
| | | JP | 2016000219 A | 07-01-2016 |
| | | JP | 2018126564 A | 16-08-2018 |
| | | US | 2012071870 A1 | 22-03-2012 |
| | | US | 2014058197 A1 | 27-02-2014 |
| | | WO | 2011143468 A2 | 17-11-2011 |
| US 2023414274 A1 | 28-12-2023 | US | 2023414274 A1 | 28-12-2023 |
| | | WO | 2022104158 A2 | 19-05-2022 |
| US 2016192981 A1 | 07-07-2016 | AU | 2010319477 A1 | 24-05-2012 |
| | | CA | 2780608 A1 | 19-05-2011 |
| | | CN | 102711645 A | 03-10-2012 |
| | | CN | 106618731 A | 10-05-2017 |
| | | EP | 2498705 A1 | 19-09-2012 |
| | | EP | 2842510 A1 | 04-03-2015 |
| | | EP | 4111995 A1 | 04-01-2023 |
| | | JP | 6000851 B2 | 05-10-2016 |
| | | JP | 6734082 B2 | 05-08-2020 |
| | | JP | 2013510676 A | 28-03-2013 |
| | | JP | 2016152926 A | 25-08-2016 |
| | | JP | 2018187407 A | 29-11-2018 |
| | | KR | 20120101007 A | 12-09-2012 |
| | | US | 2012310233 A1 | 06-12-2012 |
| | | US | 2016192981 A1 | 07-07-2016 |
| | | US | 2018042668 A1 | 15-02-2018 |
| | | US | 2020085495 A1 | 19-03-2020 |
| | | US | 2022370123 A1 | 24-11-2022 |
| | | WO | 2011060200 A1 | 19-05-2011 |

EPO FORM P0459

For more details about this annex : see Official Journal of the European Patent Office, No. 12/82

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- WO 2018191149 A
- US 8295902 B
- CN 112545643
- US 8992519 B
- US 9060756 B
- US 5443489 A
- US 5480422 A
- US 5546951 A
- US 5568809 A
- US 5391199 A
- US 10653480 B